# EUROPÄISCHE PATENTSCHRIFT

(11) **EP 2 415 489 B1**
(45) Veröffentlichungstag und Bekanntmachung des Hinweises auf die Patenterteilung: **06.07.2016**
(21) Anmeldenummer: 11173731.8
(22) Anmeldetag: 13.07.2011
(51) Int. Cl.: A61L 31/02, A61L 31/10, A61L 31/14

(54) **Polylactid-beschichtetes Implantat aus einer biokorrodierbaren Magnesiumlegierung**
Polylactide-coated implant composed of a biocorrodible magnesium alloy
Implant revêtu d'agents polylactiques issu d'un alliage de magnésium biocorrodable

(30) Priorität: 03.08.2010 US 370101 P
(43) Veröffentlichungstag der Anmeldung: 08.02.2012
(73) Patentinhaber: Biotronik AG, 8180 Bülach (CH)
(72) Erfinder: Klocke, Björn, 8006 Zürich (CH); Borck, Alexander, 91086 Aurachtal (DE)
(74) Vertreter: Randoll, Sören

(56) Entgegenhaltungen:
- EP-A1- 1 977 722
- EP-A1- 2 116 576
- EP-A2- 2 289 575
- WO-A2-2008/089434
- US-A1- 2009 248 147

## Beschreibung

Die Erfindung betrifft ein Implantat mit einem Grundkörper aus einer biokorrodierbaren Magnesiumlegierung und einer Beschichtung aus Polylactid.

### Stand der Technik und Hintergrund der Erfindung

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, z.B. Stents), zur Befestigung und temporären Fixierung von Gewebeimplantaten und Gewebstransplantationen, aber auch zu orthopädischen Zwecken, zum Beispiel als Nagel, Platte oder Schraube. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die vorrangig zur Abdichtung des Aneurysmas dienen. Die Stützfunktion ist zusätzlich gegeben.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Das Implantat, insbesondere der Stent, besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder Ti-A16Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Die vorliegende Erfindung befasst sich mit biokorrodierbaren Basislegierungen des Magnesiums.

Bekannt ist ferner, dass sich ein höheres Maß an Biokompatibilität erreichen lässt, wenn Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen. Die Wirkstoffe können zum Beispiel auf der Implantatoberfläche in Form einer Beschichtung bereitgestellt werden.

Der Einsatz biokorrodierbarer Magnesiumlegierungen für temporäre Implantate mit filigranen Strukturen ist insbesondere dadurch erschwert, als dass die Degradation des Implantats in vivo sehr rasch abläuft. Um die Korrosionsrate, d. h. die Abbaugeschwindigkeit, zu mindern, sind verschiedene Ansätze in der Diskussion. Zum Einen wird versucht, auf Seiten des Implantatswirkstoffs durch entsprechende Legierungsentwicklung die Degradation zu verlangsamen. Zum Anderen sollen Beschichtungen eine temporäre Hemmung des Abbaus bewirken. Letzterer Ansatz erfordert demnach, dass die Beschichtung für einen reproduzierbaren Zeitraum die Korrosion verhindert oder hemmt, dann jedoch rasch eine vollständige Auflösung des Implantats ermöglicht. <hier Seite 3a einfügen>

### Zusammenfassung der Erfindung

Ein oder mehrere der zuvor angesprochenen Nachteile des Standes der Technik werden mit Hilfe des erfindungsgemäßen Implantats mit einem Grundkörper aus einer biokorrodierbaren Magnesiumlegierung und einer Beschichtung aus Polylactid gelöst oder zumindest gemindert. Das Implantat zeichnet sich dadurch aus, dass die Beschichtung als Additive Impfkristalle und lipophile Substanzen enthält.

EP2289575 A2 offenbart ein medizinisches Implantat umfassend ein Antioxidant. EP 1977722 A1 offenbart ein Stent mit einer polymeren Beschichtung. In WO 2008/089434 A2 wird ein polymerer Stent beschrieben. In EP 2116576 A1 wird eine Zusammensetzung auf Basis von Polylactid offenbart.

Der Erfindung liegt die Erkenntnis zugrunde, dass sich die Korrosion von Implantaten aus biokorrodierbaren Magnesiumlegierungen dadurch zeitlich hinauszögern lässt, dass eine Beschichtung auf Basis eines Polylactids aufgebracht wird, die sich entweder dadurch auszeichnet, dass das Polymer in einem feindispersen, kristallinen Zustand vorliegt, oder bei der die Beschichtung hydrophobe Additive enthält. Durch diese Maßnahmen wird die Beschichtung undurchlässiger für das Korrosionsmedium. Vorzugsweise wird Poly-L-lactid oder Poly-D-Lactid verwendet.

Zur Beeinflussung der Kristallinität des Polymers im erfindungsgemäßen Sinne werden Impfkristalle zugesetzt. Die Morphologie des polymeren Materials wird auf diese Weise derart beeinflusst, dass sich eine Vielzahl kleinster kristalliner Domänen bildet, die homogen über das polymere Material verteilt sind. Als Impfkristalle werden vorzugsweise Mikropartikel auf Basis von insbesondere Asche, Graphit, Tonminerale oder Silikaten (insbesondere Kaolin) eingebracht, an denen die Kristallbildung des Polymers initiiert wird. Durch die Feinstverteilung von diesen Impfkristallen ist es möglich, feinste kristalline Domänen zu erzeugen, die zudem homogen über das Polymer verteilt sind. Hierdurch kann erreicht werden, dass der kristalline Anteil im Polymer gegenüber herkömmlich aufgetragenen Polymerschichten erhöht ist, was wiederum zu einer erhöhten Dichte des Materials und verringerter Wasseraufnahme und geringerem Quellverhalten führt. Die geringere Wassermenge in kristallinen Polymeren ist für die größere Stabilität durch geringeren hydrolytischen Abbau verantwortlich. Der Anteil amorpher/kristalliner Domänen ist materialunabhängig und wird durch Parameter wie Polymerart, Symmetrie, Polymerisationsgrad und Polydispersität determiniert, das heißt bei einem gegebenen Material ist der kristalline Anteil vorgegeben, wenn das Material in thermodynamisch stabilen Zustand vorliegt.

Insbesondere wenn die polymere Matrix wirkstoffbeladen ist, wird die Fähigkeit des Materials, kristalline Bereiche auszubilden, vermindert. Durch das Erzeugen einer besonders kompakten, wenig quellenden Polylactidschicht mit hohem Kristallisationsanteil kann dem entgegengewirkt werden. Die Impfkristalle werden vorzugsweise mit einem Gewichtsanteil von 0,01 - 2%, insbesondere 0,2 - 1% bezogen auf das Gesamtgewicht des Polymers zugesetzt.

Ein zweiter Ansatz besteht darin, dem Polymer eine lipophile Substanz zuzusetzen und damit die Hydrophobie des polymeren Materials insgesamt zu erhöhen. Hierdurch kann die Wasseraufnahme des Polymers und Quellung vermindert werden. Insbesondere eigenen sich hierzu Squalen, Squalan oder Cholesterin. Vorzugsweise beträgt ein Gewichtsanteil der lipophilen Substanzen 0,5 - 10 Gew.%, insbesondere 1 - 4 Gew.% bezogen auf das Gesamtgewicht des Polymers.

Die lipophilen Substanzen haben vorzugweise einen logP-Wert von > -0.5. Der P-Wert oder auch Octanol/Wasser-Verteilungskoeffizient ist ein dimensionsloser Verteilungskoeffizient, der das Verhältnis der Konzentrationen einer Chemikalie in einem Zweiphasensystem aus 1-Octanol und Wasser angibt (bei 25°C). P wird in der Regel in der Form des dekadischen Logarithmus als Log P angegeben.

Eine Beschichtung im Sinne der Erfindung ist eine zumindest abschnittsweise Auftragung der Komponenten der Beschichtung auf den Grundkörper des Implantats. Vorzugsweise wird die gesamte Oberfläche des Grundkörpers des Implantats von der Beschichtung bedeckt. Eine Schichtdicke liegt vorzugsweise im Bereich von 1 nm bis 100 µm, besonders bevorzugt 300 nm bis 15 µm. Die erfindungsgemäße Beschichtung kann direkt auf die Implantatoberfläche aufgetragen werden oder es sind weitere Zwischenschichten vorgesehen; so kann gegebenenfalls der Grundkörper des Implantats eine anorganische Grundschicht aufweisen, die die Haftung der erfindungsgemäßen Beschichtung verbessert. Auf die erfindungsgemäße Beschichtung können weitere Schichten aufgetragen werden (zum Beispiel so genannte top coat - Schichten). Verfahren zur Beschichtung von Implantaten sind dem Fachmann bekannt.

Als biokorrodierbar im Sinne der Erfindung werden Legierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Unter Magnesiumlegierung wird vorliegend ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C und pH 7,38 gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Eine besonders geeignete Legierung auf Basis einer Magnesiumlegierung hat folgende Zusammensetzung: Magnesium: > 90% Yttrium: 3,7% - 5,5% Seltene Erden (ohne Yttrium): 1,5% - 4,4% Rest: < 1 % wobei sich die Prozentangaben auf Gew.% beziehen. Vorzugsweise umfasst die Formulierung ferner eine Magnesiumlegierung mit einem Gehalt von Yttrium im Bereich von 3,7 - 5,5 Gew.%, einem Gehalt von Neodym im Bereich von 1,8 - 2,7 Gew.% und einem Gehalt von Zirkonium im Bereich von 0,2 - 1,2 Gew.%. Besonders bevorzugt entspricht die Formulierung der kommerziell erhältlichen Magnesiumlegierung WE43. Die vorgenannten Materialien und Angaben zur Zusammensetzung zeichnen sich durch ihre gute Verarbeitbarkeit und günstiges Freisetzungsverhalten für Magnesium beim in vivo-Abbau des Trägers aus. Aus der Literatur ist u.a. eine Studie zum Degradationsverhalten einer Magnesiumlegierung unter physiologischen Bedingungen bekannt, die Hinweise dazu liefert, welche Faktoren und Maßnahmen bei der Optimierung der Wirkstofffreisetzung zu beachten sind (Levesque, J., Dube, D., Fiset M. and Mantovani, D. (2003) Material Science Forum Vols. 426-432, pp. 225-238).

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht hier aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Salinität, Volumen-Oberflächen-Verhältnis, Konzentrationsunterschied sowie Strömungsgeschwindigkeit beeinflusst.

An der Phasengrenzfläche zwischen Werkstoff und Medium laufen Redox-Reaktionen ab. Für eine schützende bzw. hemmende Wirkung müssen vorhandene Schutzschichten und/oder die Produkte der Redox-Reaktionen eine gegen das Korrosionsmedium ausreichend dichte Struktur ausbilden, eine bezogen auf die Umgebung erhöhte thermodynamische Stabilität aufweisen und im Korrosionsmedium wenig löslich oder unlöslich sein. In der Phasengrenzfläche, genauer in einer sich in diesem Bereich ausbildenden Doppelschicht, laufen Ad- und Desorptionsprozesse ab. Die Vorgänge in der Doppelschicht sind geprägt von den dort ablaufenden Transport- und Diffusionsprozessen. Bei Magnesiumlegierungen ist in der Regel eine allmähliche Alkalisierung der Doppelschicht zu beobachten. Fremdstoffablagerungen, Verunreinigungen und Korrosionsprodukte beeinflussen den Korrosionsprozess. Die Vorgänge bei der Korrosion, insbesondere der Geschwindigkeitsbestimmende Schritt, sind demnach hoch komplex und lassen sich gerade im Zusammenhang mit einem physiologischen Korrosionsmedium, also Blut oder künstlichem Plasma, nicht oder nur im geringen Umfang voraussagen, da Vergleichsdaten fehlen. Schon aus diesem Grunde ist das Auffinden einer korrosionshemmenden Beschichtung, d. h. einer Beschichtung, die nur zur temporären Herabsetzung der Korrosionsrate eines metallischen Werkstoffs der weiter oben genannten Zusammensetzung in physiologischer Umgebung dient, eine außerhalb der Routine eines Fachmanns liegende Maßnahme. Dies gilt insbesondere für Stents, welche bei der Implantation lokal hohen plastischen Verformungen ausgesetzt sind. Konventionelle Ansätze mit starren korrosionshemmenden Schichten sind unter derartigen Voraussetzungen ungeeignet.

Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Formkörpers wird ein im Sinne der Erfindung beschichtetes Implantat zur Herabsetzung der Korrosionsrate führen. Die erfindungsgemäße korrosionshemmende Beschichtung kann im Laufe der Zeit selbst abgebaut werden bzw. kann die von ihr abgedeckten Bereiche des Implantats nur noch in einem immer geringeren Umfang schützen. Daher ist der Verlauf der Korrosionsrate für das gesamte Implantat nicht linear. Vielmehr ergibt sich zu Beginn der einsetzenden korrosiven Prozesse eine relativ niedrige Korrosionsrate, die im Laufe der Zeit ansteigt. Dieses Verhalten wird als temporäre Herabsetzung der Korrosionsrate im Sinne der Erfindung verstanden und zeichnet die korrosionshemmende Beschichtung aus. Im Falle von Koronarstents soll die mechanische Integrität der Struktur über einen Zeitraum von drei bis sechs Monaten nach Implantation aufrechterhalten werden.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu beschichten. Vorzugsweise ist das Implantat ein Stent.

Die polymere Matrix kann einen oder mehrere Wirkstoffe enthalten, der nach der Implantation freigesetzt wird. Der Wirkstoff kann in das polymere Material eingebettet sein.

Nach einer weiteren bevorzugten Ausführungsform enthält die Beschichtung Partikel, die aus Polylactid bestehen und einem Additiv (Impfkristalle oder lipophile Substanz), jedoch keine Wirkstoffe enthalten.

Vorzugsweise werden Schichten mit Wirkstoff ergänzend außen auf das Implantat aufgebracht, das bereits mit einer korrosionsverlangsamenden Schicht aus polymerer Matrix und Zusätzen behandelt wurde, dass heißt, zusätzlich sind eine oder mehrere wirkstoffhaltige Schichten auf der Beschichtung aus Polylactid und/oder dem Implantatgrundkörper aufgebracht. Die zusätzlichen, wirkstoffhaltigen Schichten können dabei entweder vollständig und teilweise, bei einem Stent vorzugsweise abluminal, die Implantatoberfläche bedecken.

Unter Wirkstoff im erfindungsgemäßen Sinne wird ein Arzneistoff mit pharmazeutischer Wirkung verstanden, der im menschlichen oder tierischen Körper zur Heilung, Linderung, Verhütung oder Erkennung von Krankheiten dient. Wirkstoffe umfassen insbesondere Paclitaxel, Sirolimus/Rapamycin und deren Derivate und Pro-Drugs. Insbesondere sind Wirkstoffe vorteilhaft, die auf mTOR wirken, sowie RAS Inhibitoren, insbesondere solche, die die RAS-Adhäsion verhindern. Auch antiinflammatorische und antithrombogene Wirkstoffe sind von Vorteil.

Bevorzugt ist ferner ein Implantat (insbesondere Stent), dessen Stützzeit durch die Beschichtung aus Polylactid in vivo um 2 Wochen bis 6 Monate gegenüber einem unbeschichteten Implantat erhöht ist.

### Detaillierte Beschreibung der Erfindung

Die Erfindung wird nachfolgend anhand von Beispielen näher erläutert.

### Vergleichsbeispiel 1

2 g Poly-L-Lactid (PLLA), 350 mg Rapamycin und 40 mg Squalen werden in ca. 11 CHCl₃ gelöst. Die Lösung wird über ein herkömmliches Sprühverfahren auf einen Stent aus einer biokorrodierbaren Magnesiumlegierung aufgetragen.

### Vergleichsbeispiel 2

2 g Poly-L-Lactid (PLLA), 350 mg Rapamycin und 10 mg Kaolin (feiner Mahlgrad; < 0.5 µm) werden in ca. 1 1 CHCl₃ dispergiert. Die Suspension wird über ein Sprühverfahren auf einen Stent aus einer biokorrodierbaren Magnesiumlegierung aufgetragen.

### Vergleichsbeispiel 3

Eine erste Beschichtung erfolgt gemäß Ausführungsbeispiel 1 oder 2, jedoch ohne Zusatz von Rapamycin. Der erhaltene beschichtete Stent wird mit einer weiteren Beschichtungslösung, die PLLA und Rapamycin im Gewichtsverhältnis 1:1 in CHCl₃ enthält, bei 50°C mit einem Abstand von 30 cm auf eine PTFE Platte angesprüht. Temperatur und Abstand führen zu einer Sprühtrocknung; die erhaltenen Partikel werden in die noch feuchte erste Beschichtung eingetragen/aufgebracht. Optional kann eine weitere Deckschicht aufgetragen werden.

## Patentansprüche

1. Implantat mit einem Grundkörper aus einer biokorrodierbaren Magnesiumlegierung und einer Beschichtung aus Polylactid, wobei die Beschichtung als Additive Impfkristalle und lipophile Substanzen enthält.

2. Implantat nach Anspruch 1, bei dem die Impfkristalle ausgewählt sind aus der Gruppe umfassend Asche, Graphit, Silikate und Tonminerale.

3. Implantat nach Anspruch 1, bei dem das Polylactid aus Poly-L-lactid aufgebaut ist.

4. Implantat nach Anspruch 1, bei dem das Polylactid aus Poly-D-lactid aufgebaut ist.

5. Implantat nach Anspruch 1 oder 2, bei dem die lipophilen Substanzen einen logPWert von größer als -0.5 aufweisen.

6. Implantat nach Anspruch 3, bei dem die lipophile Substanz Squalen, Squalan oder Cholesterin ist.

7. Implantat nach einem der vorhergehenden Ansprüche, bei dem die Beschichtung einen Wirkstoff enthält.

8. Implantat nach einem der vorhergehenden Ansprüche, bei dem das Implantat ein Stent ist.

9. Implantat nach einem der Ansprüche 1 bis 6 und 8, bei dem die Beschichtung Partikel enthält, die aus Polylactid bestehen und einem Additiv nach Anspruch 1, jedoch keine Wirkstoffe enthalten.

10. Implantat nach einem der vorhergehenden Ansprüche, bei dem die Impfkristalle mit einem Gewichtsanteil von 0,01 - 2% bezogen auf das Gesamtgewicht des Polymers aufweisen.

11. Implantat nach einem der vorhergehenden Ansprüche, bei dem die lipophilen Substanzen einen Gewichtsanteil von 0,5 - 10 Gew.% bezogen auf das Gesamtgewicht des Polymers aufweisen.

12. Implantat nach einem der vorhergehenden Ansprüche, bei dem zusätzlich eine oder mehrere wirkstoffhaltige Schichten auf der Beschichtung aus Polylactid und/oder dem Implantatgrundkörper aufgebracht sind.

13. Implantat nach einem der vorhergehenden Ansprüche, dessen Stützzeit durch die Beschichtung aus Polylactid in vivo um 2 Wochen bis 6 Monate erhöht ist.

## Claims

1. An implant having a main body composed of a biocorrodible magnesium alloy and a polylactide coating, wherein the coating contains seed crystals and lipophilic substances as additives.

2. The implant according to claim 1, wherein the seed crystals are selected from the group comprising ash, graphite, silicates, and clay minerals.

3. The implant according to claim 1, wherein the polylactide is formed from poly-L-lactide.

4. The implant according to claim 1, wherein the polylactide is formed from poly-D-lactide.

5. The implant according to claim 1 or 2, wherein the lipophilic substances have a log P value of greater than -0.5.

6. The implant according to claim 3, wherein the lipophilic substance is squalene, squalene or cholesterol.

7. The implant according to any one of the preceding claims, wherein the coating contains an active substance.

8. The implant according to any one of the preceding claims, wherein the implant is a stent.

9. The implant according to any one of claims 1 to 6 and 8, wherein the coating contains particles which consist of polylactide and an additive according to claim 1, but which do not contain any active substances.

10. The implant according to any one of the preceding claims, wherein the seed crystals have a proportion by weight of 0.01-2%, relative to the total weight of the polymer.

11. The implant according to any one of the preceding claims, wherein the lipophilic substances have a proportion by weight of 0.5-10 % by weight, relative to the total weight of the polymer.

12. The implant according to any one of the preceding claims, wherein one or more layers containing active substance is/are additionally applied to the polylactide coating and/or the main body of the implant.

13. The implant according to any one of the preceding claims, which achieves a support time that is increased by 2 weeks to 6 months *in vivo* as a result of the polylactide coating.

## Revendications

1. Implant avec un corps de base constitué d'un alliage de magnésium biocorrodable et un revêtement constitué de polylactide, où le revêtement contient en tant qu'additifs des germes cristallins et des substances lipophiles.

2. Implant selon la revendication 1, chez lequel les germes cristallins sont choisis dans le groupe comprenant de la cendre, du graphite, des silicates et des minéraux argileux.

3. Implant selon la revendication 1, chez lequel le polylactide est basé sur du poly-L-lactide.

4. Implant selon la revendication 1, chez lequel le polylactide est basé sur du poly-D-lactide.

5. Implant selon les revendications 1 ou 2, chez lequel les substances lipophiles présentent une valeur de logP supérieure à -0,5.

6. Implant selon la revendication 3, chez lequel la substance lipophile est du squalène, du squalane ou du cholestérol.

7. Implant selon l'une des revendications précédentes, chez lequel le revêtement contient une matière active.

8. Implant selon l'une des revendications précédentes, chez lequel l'implant est un stent.

9. Implant selon l'une des revendications 1 à 6 et 8, chez lequel le revêtement contient des particules qui sont constituées de polylactide et d'un additif selon la revendication 1, mais ne contient pas de matière active.

10. Implant selon l'une des revendications précédentes, chez lequel des germes cristallins se présentent avec une proportion en poids de 0,01 à 2 % par rapport au poids total du polymère.

11. Implant selon l'une des revendications précédentes, chez lequel les substances lipophiles présentent une proportion en poids de 0,5 à 10 % en poids par rapport au poids total de polymère.

12. Implant selon l'une des revendications précédentes, chez lequel en outre une ou plusieurs couches contenant de la substance active sont rapportées sur le revêtement de polylactide et/ou sur le corps de base d'implant.

13. Implant selon l'une des revendications précédentes, dont la durée de maintien in vivo est augmentée de 2 semaines à 6 mois par le revêtement de polylactide.
